Europäisches Patentamt

European Patent Office

Office européen des brevets

(19)

(11) Numéro de publication: **0 075 522**
**B1**

(12) **FASCICULE DE BREVET EUROPEEN**

(45) Date de publication du fascicule du brevet:
**27.12.85**

(51) Int. Cl.⁴: **A 61 K 35/80**

(21) Numéro de dépôt: **82401720.6**

(22) Date de dépôt: **23.09.82**

(54) Nouveaux médicaments à base d'extraits d'algues, et formulations correspondantes.

(30) Priorité: **23.09.81 FR 8117943**

(43) Date de publication de la demande:
**30.03.83 Bulletin 83/13**

(45) Mention de la délivrance du brevet:
**27.12.85 Bulletin 85/52**

(84) Etats contractants désignés:
**AT BE CH DE GB IT LI LU NL SE**

(56) Documents cités:
**AU - A - 48 622**
**BE - A - 693 094**
**FR - A - 1 057 844**
**FR - A - 2 242 991**

**BIOLOGICAL ABSTRACTS, vol. 68, no. 3, 1979, no. 177441, Philadelphia, Pennsylvania (USA); N.Z. ZHILTSOV et al.: "Marine organisms as a source of biologically active substances".**
**DUVAL et DUVAL, Dictionnaire de Chimie, p. 773**

(73) Titulaire: **LABORATOIRES GOEMAR S.A., Avenue du Général Patton Boîte Postale 55, F-35403 Saint-Malo (FR)**

(72) Inventeur: **Herve, René, Les Tertres Route de Lamballe Pluduno, F-22130 Plancoet (FR)**
Inventeur: **Percehais, Serge, 8, rue d'Orleans, F-35400 Saint Malo (FR)**

(74) Mandataire: **Ranguis, Patrick Frédéric et al, Cabinet Beau de Loménie 55, rue d'Amsterdam, F-75008 Paris (FR)**

**Description**

La présente invention concerne l'utilisation en thérapeutique d'extraits de certaines algues préparés d'une manière spéciale.

On connait déjà par AU-A-48 622 l'utilisation par voie externe de poudre d'algues brunes associée à de l'iode et, par BE-A-693 094 l'usage également externe de poudre d'algues broyées à chaud, associée à de l'eau de mer.

On sait qu'il existe plusieurs familles d'algues.

Les algues qui sont utilisées selon la présente invention appartiennent à la famille des algues brunes ou phéophycées et des algues rouges ou rodophycées.

L'invention vise plus particulièrement la découverte de propriétés surprenantes des algues suivantes:

— algues brunes:
  — Bifurcaria Rotunda
  — Fucus Vesiculosus
  — Ascophyllum Nodosum
  — Pelvetia Canaliculata
— algues rouges:
  — Delesseria Sanguinea

Les propriétés découvertes selon l'invention sont étroitement liées au traitement spécial de ces algues qui conduit à un produit présentant des caractéristiques particulières physicochimiques.

Les algues sont traitées de la manière suivante.

On récolte les algues, et on les lave en piscine afin de les débarrasser des animacules et du sable.

On leur fait ensuite subir une surgélation, notamment dans un surgélateur à plaques, à −10/−30°C, afin de permettre la conservation des éléments utiles.

On effectue ensuite en général une conservation en chambre froide en raison du fait que les récoltes sont saisonnières.

Les algues sont ensuite soumises à un cryobroyage (on peut utiliser deux broyeurs en cascade sous azote liquide) puis à un laminage (on peut utiliser une machine à cylindres) et enfin à une homogénéisation. On obtient ainsi une »bouillie-mère« dont les particules constitutives présentent une dimension de 6 à 20 μm environ. Cette bouillie-mère est aussi appelée »crème d'algues«.

On peut faire passer cette bouillie-mère sur une décanteuse à grande vitesse qui donne deux produits, d'une part le gâteau que l'on dénomme »base d'algues« et d'autre part le jus de décantation que l'on dénomme »protoexoplasma d'algues«.

Egalement à titre indicatif, il est possible d'effectuer une lyophilisation aussi bien de la crème d'algues que du protoexoplasma d'algues pour donner une forme sèche qui peut donner lieu par exemple à la fabrication de comprimés, etc.

Cette suite d'opérations est bien entendu valable pour chaque variété d'algues.

Dans le domaine de la préparation de ces différents extraits, on pourra se référer utilement au brevet français n° 74/35 162 déposé le 18 octobre 1974 au nom de HERVE et ROULLIER.

Le gâteau d'algues permet de fabriquer par exemple des savons et analogues, car il contient environ 4 à 6% de cellulose, 38 à 40% d'alginates insolubles, ainsi que des vitamines liposolubles.

Cependant, la présente invention concerne l'utilisation aux fins précitées du »protoexoplasma« d'algues.

L'invention concerne des médicaments pour le traitement des carences en oligo-élément et administrés par voie orale, caractérisés en ce qu'ils contiennent:

— un »protoexoplasma« en jus obtenu par décantation à grande vitesse d'une bouillie mère, elle même obtenue par cryobroyage d'une algue brune on rouge, et
— au moins un composé de l'oligo-élément dont on a constaté la carence.

Les essais effectués ont mis en évidence pour ces produits le fait qu'ils forment un véhicule qui favorise l'assimilation d'oligo-éléments tels que Cu, Mg, Mn et Zn, et analogues.

Ainsi, la présente invention vise la combinaison du protoexoplasma de rodophycées ou de phéophycées et d'oligo-éléments, présentant comme propriété surprenante le fait que l'assimilation des oligo-éléments est notablement favorisée.

Des essais ont été effectués sur un patient. L'administration de protoexoplasma seul n'a conduit à aucune augmentation du taux de magnésium dans le sang. Par contre, une administration durant un mois de 5 ml, chaque jour, matin et soir, par voie orale, d'une combinaison de protoexoplasma d'Ascophyllum Nodosum et de la concentration habituelle (30−100 mg de cations Mg par prise) en oligo-elément Mg a conduit à une augmentation du taux de magnésium dans le sang de 20‰ à 25‰ au bout d'un mois.

L'arrêt du traitement pendant un mois a permis un retour au taux initial de 20‰.

Un nouveau traitement d'un mois a fait remonter le taux de magnésium dans le sang jusqu'à 25‰.

Ainsi, en ce qui concerne le magnésium, on a observé une assimilation plus élevée qu'avec les médicaments classiques connus.

On a obtenu sensiblement les mêmes résultats avec le cuivre (12−13 μmoles/l → 14 μmoles/l).

Les nouveaux médicaments selon l'invention sont donc utiles dans les conditions précitées pour traiter les carences en oligo-éléments, notamment Cu et Mg, et permettent donc de prévenir certains accidents cardiaques notamment, et de traiter efficacement les états pathologiques connus du praticien et liés à une telle carence.

### Revendications par les états contractants BE, CH, DE, GB, IT, LI, LU, NL, SE

1. Médicaments pour la traitement des carences en oligo-éléments et administrés par voie orale, caractérisés en ce qu'ils contiennent:

— un »protoexoplasma« ou jus obtenu par décantation à grande vitesse d'une bouillie mère, elle-même obtenue par cryobroyage d'une algue brune ou rouge, et
— au moins un composé de l'oligo-élément dont on a constaté la carence.

2. Médicaments selon la revendication 1, caractérisés en ce que le protoexoplasma présente une dimension de particules d'environ 6 à 20 µm.

3. Médicaments selon la revendication 1 ou 2, caractérisés en ce que les algues utilisées pour fabriquer le protoexoplasma sont choisies parmi les suivantes:

— algues brunes:
  — Bifurcaria Rotunda
  — Fucus Vesiculosus
  — Ascophyllum Nodosum
  — Pelvetia Canaliculata

— algues rouges:
  — Delesseria Sanguinea

4. Médicaments selon l'une quelconque des revendications 1 à 3, caractérisé en ce que l'algue utilisée est l'Ascophyllum Nodosum.

5. Médicaments selon l'une quelconque des revendications 1 à 4, caractérisés en ce que les oligo-éléments sont choisis parmi Cu, Mg, Mn, Zn.

6. Forme pharmaceutique d'administration par voie orale des médicaments selon l'une quelconque des revendications 1 à 5, sous forme orale unitaire de 5 ml.

### Revendications pour l'état contractant AT

1. Utilisation d'un »protoexoplasma« ou jus obtenu par décantation à grande vitesse d'une bouillie mère, elle-même obtenue par cryobroyage d'une algue brune ou rouge, et d'au moins un composé de l'oligo-élément dont on a constaté la carence, pour la fabrication d'un médicament administrable par voie orale, utile pour le traitement des carences en oligo-éléments.

2. Utilisation selon la revendication 1, caractérisée en ce que le protoexoplasma présente une dimension de particules d'environ 6 à 20 µm.

3. Utilisation selon la revendication 1 ou 2, caractérisée en ce que les algues utilisées pour fabriquer le protoexoplasma sont choisies parmi les suivantes:

— algues brunes:
  — Bifurcaria Rotunda
  — Fucus Vesiculosus
  — Ascophyllum Nodosum
  — Pelvetia Canaliculata

— algues rouges:
  — Delesseria Sanguinea

4. Utilisation selon l'une quelconque des revendications 1 à 3, caractérisée en ce que l'algue utilisée est l'Ascophyllum Nodosum.

5. Utilisation selon l'une quelconque des revendications 1 à 4, caractérisée en ce que les oligo-éléments sont choisis parmi Cu, Mg, Mn, Zn.

6. Utilisation selon une forme pharmaceutique d'administration par voie orale unitaire de 5 ml d'un médicament obtenu selon l'une des revendications 1 à 5.

### Patentansprüche für die Vertragsstaaten: BE, CH, DE, GB, IT, LI, LU, NL, SE

1. Neue Medikamente, die insbesondere für die Behandlung von Mängeln an Spurenelementen nützlich sind, dadurch gekennzeichnet, daß sie aus der Kombination eines »Protoexoplasmas« von Braun- oder Rotalgen und den jeweiligen Spurenelementen bestehen.

2. Neue Medikamente nach Anspruch 1, dadurch gekennzeichnet, daß das Protoexoplasma eine Teilchengröße von etwa 6 bis 20 µ aufweist.

3. Neue Medikamente nach Anspruch 1 oder 2, dadurch gekennzeichnet, daß die zur Herstellung des Protoexoplasmas verwendeten Algen aus den folgenden ausgewählt sind:

— Braunalgen:
  — Bifurcaria Rotunda
  — Fucus Vesiculosus
  — Ascophyllum Nodosum
  — Pelvetia Canaliculata

— Rotalgen:
  — Delesseria Sanguinea.

4. Neue Medikamente nach irgendeinem der Ansprüche 1 bis 3, dadurch gekennzeichnet, daß die verwendete Alge Ascophyllum Nodosum ist.

5. Neue Medikamente nach irgendeinem der Ansprüche 1 bis 4, dadurch gekennzeichnet, daß die Spurenelemente ausgewählt sind aus Cu, Mg, Mn, Zn.

6. Pharmazeutische Form zur oralen Verabreichung der neuen Medikamente nach irgendeinem der Ansprüche 1 bis 5 in oraler Einheitsform von 5 ml.

### Patentansprüche für den Vertragsstaat: AT

1. Verwendung eines durch Dekantieren bei hoher Geschwindigkeit aus einer Mutterbrühe erhaltenen »Protoexoplasma« oder Saftes, wobei die Mutterbrühe durch Kryozerkleinerung einer Braun- oder Rotalge erhalten wurde, und mindestens einer Verbindung des Spurenelementes, dessen Mangel festgestellt worden ist, zur Herstellung eines oral verabreichbaren Medikamentes, das für die Behandlung von Mängeln an Spurenelementen nützlich ist.

2. Verwendung nach Anspruch 1, dadurch gekennzeichnet, daß das Protoexoplasma eine Teilchengröße von etwa 6 bis 20 µm aufweist.

3. Verwendung nach Anspruch 1 oder 2, dadurch gekennzeichnet, daß die zur Herstellung des Protoexoplasmas verwendeten Algen aus den folgenden ausgewählt sind:

— Braunalgen:
  — Bifurcaria Rotunda
  — Fucus Vesiculosus
  — Ascophyllum Nodosum
  — Pelvetia Canaliculata

— Rotalgen:
  —Delesseria Sanguinea.

4. Verwendung nach irgendeinem der Ansprüche 1 bis 3, dadurch gekennzeichnet, daß die verwendete Alge Ascophyllum Nodosum ist.

5. Verwendung nach irgendeinem der Ansprüche 1 bis 4, dadurch gekennzeichnet, daß die Spurenelemente ausgewählt sind aus Cu, Mg, Mn, Zn.

6. Verwendung eines nach einem der Ansprüche 1 bis 5 erhaltenen Medikamentes in einer pharmazeutischen Form zur oralen Verabreichung in Einheiten von 5 ml.

## Claims for the contracting States: BE, CH, DE, GB, IT, LI, LU, NL, SE

1. Novel drugs particularly useful in the treatment of deficiencies in trace elements, characterized in that they consist in combining a »protoexoplasma« of brown or red algae with trace elements in question.

2. Novel drugs according to claim 1, characterized in that the protoexoplasma has a particle size of about 6 to 20 µ.

3. Novel drugs according to claim 1 or 2, characterized in that the algae used to prepare the protoexoplasma are selected from the following:

— brown algae:
  — Bifurcaria Rotunda
  — Fucus Vesiculosus
  — Ascophyllum Nodosum
  — Pelvetia Canaliculata

— red algae:
  — Delesseria Sanguinea

4. Novel drugs according to any one of claims 1 to 3, characterized in that the algae used is Ascophyllum Nodosum.

5. Novel drugs according to any one of claims 1 to 4, characterized in that the trace elements are selected from Cu, Mg, Mn, Zn.

6. Pharmaceutical form of administration by the oral route of novel drugs according to any one of claims 1 to 5, in oral unit form of 5 ml.

## Claims for the Contracting State: AT

1. Use of a »protoexoplasma« or liquor obtained by decantation at high speed of a mother pulp, which is itself obtained by cryogrinding of a brown or red algae, and at least one compound of the trace element of which a deficiency has been noted, for the preparation of a drug to be administered by the oral route useful for the treatment of deficiencies of trace elements.

2. Use according to claim 1, characterized in that the protoexoplasma has a particle size of about 6 to 20 µm.

3. Use according to claim 1 or 2, characterized in that the algae used to prepare the protoexoplasma are selected from the following:

— brown algae:
  — Bifurcaria Rotunda
  — Fucus Vesiculosus
  — Ascophyllum Nodosum
  — Pelvetia Canaliculata

— red algae:
  — Delesseria Sanguinea

4. Use according to any one of claims 1 to 3, characterized in that the algae used is Ascophyllum Nodosum.

5. Use according to any one of claims 1 to 4, characterized in that the trace elements are selected from Cu, Mg, Mn, Zn.

6. Use in a pharmaceutical form of unitary administration by the oral route of 5 ml of a drug obtained according to any one of claims 1 to 5.